# EUROPÄISCHE PATENTSCHRIFT

(11) **EP 2 913 034 B1**
(45) Veröffentlichungstag und Bekanntmachung des Hinweises auf die Patenterteilung: **07.08.2019**
(21) Anmeldenummer: 14156968.1
(22) Anmeldetag: 27.02.2014
(51) Int. Cl.: A61F 9/007

(54) **Chirurgisches Handstück**
Surgical hand-held tool
Pièce à main chirurgicale

(43) Veröffentlichungstag der Anmeldung: 02.09.2015
(73) Patentinhaber: EOS GmbH, 52249 Eschweiler (DE)
(72) Erfinder: Klomp, Manfred, 4191AA Geldermalsen (NL)
(74) Vertreter: Schwarz & Partner Patentanwälte OG

(56) Entgegenhaltungen:
- WO-A1-02/41788
- WO-A1-98/52502
- WO-A1-2013/009576
- US-A1- 2010 106 054

## Beschreibung

Chirurgisches Handstück zum Entfernen von biologischem Gewebe umfassend einen hohlen, äußeren Zylinder der an einem Ende geschlossen ist, wobei im Bereich des Endes an der Mantelfläche des äußeren Zylinders ein Schneidfenster ausgebildet ist und einen hohlen, inneren Zylinder der an einem ersten und an einem zweiten Ende eine Öffnung aufweist und im äußeren Zylinder mit einem Antrieb, zwischen zwei Endpositionen, koaxial verschiebbar ist, wobei bei einem ersten Betriebsmodus eine Stirnfläche des ersten Endes mit dem Schneidfenster des äußeren Zylinders eine Schnittkante bildet.

Das Dokument US 2009/0281479 A1 offenbart ein Vitrektomie Gerät, nachfolgend als chirurgisches Handstück bezeichnet, mit dem der Glaskörper eines menschlichen Auges entfernt werden kann. Hierfür umfasst das chirurgische Handstück einen Handgriff an dem drei pneumatische Anschlüsse ausgebildet sind. Innerhalb des Handgriffs befinden sich zwei Druckkammern die durch einen verschiebbaren Kolben getrennt sind. Je eine Druckkammer ist mit je einem pneumatischen Anschluss verbunden. Über die pneumatischen Anschlüsse kommunizieren die Druckkammern mit einem Kompressor, dessen Druckluft den verschiebbaren Kolben in eine oszillierende Bewegung versetzt. Der dritte pneumatische Anschluss ist im Handgriff im Bereich des zweiten Endes des inneren Zylinders ausgebildet und dient zur Abfuhr von abgetrenntem Glaskörpergewebe. Der hohle, äußere Zylinder ist mit dem Handgriff fest verbunden, wobei das fest verbundene Ende des äußeren Zylinders eine Öffnung aufweist. Das dem Handgriff abgewandte Ende des äußeren Zylinders ist geschlossen, wobei im Bereich des geschlossenen Endes an der Mantelfläche des äußeren Zylinders ein Fenster vorgesehen ist. Innerhalb des äußeren Zylinders umfasst das chirurgische Handstück einen hohlen, inneren Zylinder welcher an beiden Enden eine Öffnung aufweist. Der innere Zylinder ist durch den äußeren Zylinder axial geführt und erstreckt sich vom dritten pneumatischen Anschluss bis in den Bereich des Fensters des äußeren Zylinders. Der innere Zylinder ist mit dem verschiebbaren Kolben fest verbunden und oszilliert angetrieben durch den Kolben im äußeren Zylinder zwischen zwei Endpositionen. Das Fenster des äußeren Zylinders bildet zusammen mit dem ersten Ende des inneren Zylinders eine Schnittkante. Bewegt sich der innere Zylinder in Richtung geschlossenes Ende des äußeren Zylinders, so vollzieht der innere Zylinder eine Vorwärtsbewegung. Zum Abtrennen wird das Fenster durch Rückwärtsbewegen des inneren Zylinders geöffnet und das Glaskörpergewebe wird durch das Fenster in den äußeren Zylinder eingesaugt. Der über den Kolben vorwärts bewegte innere Zylinder trennt, zusammen mit einer Fläche des Fensters, das eingesaugte Glaskörpergewebe vom Glaskörper ab. Über den hohlen, inneren Zylinder wird das abgetrennte Glaskörpergewebe zum dritten pneumatischen Anschluss abgeführt.

Für den beschriebenen Abtrennvorgang muss das chirurgische Handstück in den Augapfel eingeführt werden. Hierfür wird der Augapfel an einer Stelle geöffnet und über diese Öffnung wird das chirurgische Handstück in den Augapfel eingeführt. Der Eingriff erfordert einerseits höchste Präzision, andererseits ist es nicht weniger wichtig, dass der operative Eingriff in einer möglichst kurzen Zeitspanne vollzogen wird. Das vorstehend, erwähnte Dokument US 2009/0281479 A1 offenbart chirurgisches Handstück, welches für sehr präzise Abtrennvorgänge konzipiert ist. Diese Präzision wird durch einen geringen Abstand zwischen dem Fenster und dem geschlossenen Ende des äußeren Zylinders erreicht. Bei dem bekannten chirurgischen Handstück hat sich jedoch als Nachteil erwiesen, dass an Stellen des Glaskörpers, wo mit einer geringeren Präzision vorgegangen werden könnte, das chirurgische Handstück keinen alternativen Betriebsmodus bietet. Hierfür wäre ein Wechsel des chirurgischen Handstücks nötig, dabei besteht ein erhöhtes Infektionsrisiko des operativ geöffneten Augapfels.

Der Erfindung liegt die Aufgabe zugrunde ein chirurgisches Handstück zu schaffen, das für präzises und schnelles Operieren je nach Bedarf dem Operateur mehrere Betriebsmodi bietet. Dadurch besteht kein Bedarf für einen Wechsel des chirurgischen Handstücks und der Operationsvorgang kann schneller vollzogen werden.

Erfindungsgemäß wird diese Aufgabestellung dadurch gelöst, dass der innere Zylinder im Bereich des ersten Endes an der Mantelfläche eine erste Schneidöffnung aufweist und zum äußeren Zylinder verdrehbar ausgebildet ist, wobei eine Arretiervorrichtung vorgesehen ist, die den inneren Zylinder gegenüber dem äußeren Zylinder gegen Verdrehen sichert und wobei mit der Arretiervorrichtung ein zweiter Betriebsmodus einstellbar ist, bei dem das Schneidfenster des äußeren Zylinders und die erste Schneidöffnung des inneren Zylinders, zur Bildung zumindest einer weiteren Schnittkante, ausgerichtet sind.

Hierdurch ist der Vorteil enthalten, dass zwei Betriebsmodi des chirurgischen Handstücks eingestellt werden können. Beim ersten Betriebsmodus steht, wie beim Stand der Technik, nur eine Schnittkante zu Verfügung. Diese wird durch die Stirnseite des inneren Zylinders und dem Schneidfenster des äußeren Zylinders gebildet. In diesem ersten Betriebsmodus erfolgt der Schnitt während der innere Zylinder, in Richtung geschlossenes Ende des äußeren Zylinders, ein Vorwärtsbewegung vollzieht. Die Schnittkante wird infolgedessen an der Seite des Schneidfensters, welche dem geschlossenen Ende des äußeren Zylinders am nächsten liegt, gebildet. Da sich diese Schnittkante sehr nah am geschlossenen Ende des äußeren Zylinders befindet ermöglicht dieser erste Betriebsmodus, zu Lasten des Operationsfortschritts, präzise und feine Schnitte.

Für die Umstellung auf den ersten beziehungsweise auf den zweiten Betriebsmodus muss zuerst die Arretiervorrichtung gelöst werden, wodurch der innere Zylinder gegenüber dem äußeren Zylinder verdrehbar wird. Durch Drehung des inneren Zylinders gegenüber dem äußeren Zylinder erfolgt die Einstellung des gewünschten Betriebsmodus. Abschließend folgt die Sicherung des inneren Zylinders gegen Verdrehen durch Arretierung der Arretiervorrichtung.

In dem zweiten Betriebsmodus des chirurgischen Handstücks steht eine weitere Schnittkante zu Verfügung und infolgedessen kann vorteilhafterweise der Operationsfortschritt beschleunigt werden. Hierfür weist der innere Zylinder an der Mantelfläche eine erste Schneidöffnung auf, die im Bereich des Schneidfensters des äußeren Zylinders liegt. Zur Bildung einer weiteren Schnittkante werden, durch Verdrehen des inneren Zylinders zum äußeren Zylinder, die erste Schneidöffnung und das Schneidfenster zueinander ausgerichtet. Der zusätzliche Schnittvorgang kann, auch wie der Schnittvorgang an der Stirnfläche, während der Vorwärtsbewegung des inneren Zylinders erfolgen, oder aber auch während sich der innere Zylinder in Richtung Handgriff bewegt. Je nach Ausführungsform könnten im zweiten Betriebsmodus bis zu drei Schnittkanten zu Verfügung stehen. Wobei aber der Abstand der weiteren Schnittkanten zu dem geschlossenen Ende größer ist, wodurch die Präzision im zweiten Betriebsmodus geringer ist.

Die Erfindung wird durch die Ansprüche definiert.

Weitere vorteilhafte Ausgestaltungen des erfindungsgemäßen chirurgischen Handstücks werden im Folgenden anhand der Figuren näher erläutert.
Figur 1 zeigt ein chirurgisches Handstück im ersten Betriebsmodus in einem Längsschnitt und einer Schnittansicht A-A dargestellt.
Figur 2 zeigt einen Ausschnitt B gemäß Figur 1 in einer Schnittansicht, wobei der Schnittvorgang des ersten Betriebsmodus des chirurgischen Handstücks aus Figur 1 in zwei Schritten dargestellt ist.
Figur 3 zeigt den Ausschnitt B gemäß Figur 1 in einer Schnittansicht, wobei der Schnittvorgang des zweiten Betriebsmodus des chirurgischen Handstücks aus Figur 1 in drei Schritten dargestellt ist.
Figur 4 zeigt einen inneren und einen äußeren Zylinder eines erfindungsgemäßen chirurgischen Handstücks in einer Schnittdarstellung, bei dem am inneren Zylinder eine zweite Schneidöffnung ausgeführt ist.
Figur 5 zeigt einen inneren Zylinder eines erfindungsgemäßen chirurgischen Handstücks in einer Schnittdarstellung.

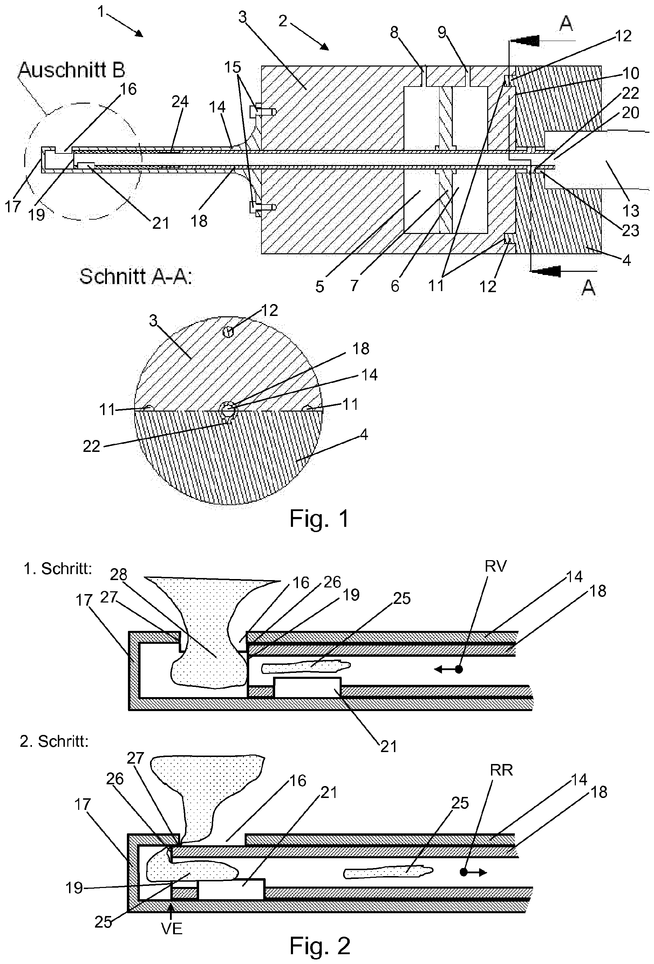

Figur 1 zeigt ein chirurgisches Handstück 1 mit einem Handgriff 2, der aus einem Hauptteil 3 und einer Arretiervorrichtung 4 besteht. Innerhalb des Hauptteils 3 sind zwei Druckkammern 5 und 6 ausgebildet, die durch einen verschiebbaren Kolben 7 getrennt sind. Die beiden Druckkammern 5 und 6 sind über zwei pneumatische Anschlüsse 8 und 9 mit einer pneumatischen Einheit verbunden. Über diese Verbindung versetzt die pneumatische Einheit den Kolben 7 im Betrieb in eine oszillierende Bewegung.

Am Hauptteil 3 sind, an einer Trennfläche 10 zur Arretiervorrichtung 4, Vertiefungen 11 ausgebildet. Des Weiteren weist die Arretiervorrichtung 4 an der Trennfläche 10 zum Hauptteil 3, Erhöhungen 12 auf, wobei die Erhöhungen 12 so ausgebildet sind, dass sie zusammen mit den Vertiefungen 11 eine axial lösbare Verdrehsicherung bilden. An der Arretiervorrichtung 4 befindet sich, zur Abfuhr von abgetrenntem, biologischem Gewebe, ein dritter pneumatischer Anschluss 13. An der gegenüberliegenden Seite des Handgriffs 2 befindet sich ein hohler, äußerer Zylinder 14, der durch Schrauben 15 mit dem Hauptteil 3 fest verbunden ist, wobei der äußere Zylinder 14 ein Schneidfenster 16 aufweist. Das Schneidfenster 16 liegt im Bereich eines geschlossenen Endes 17 des äußeren Zylinders 14. Die lösbare Verbindung durch die Schrauben 15 ermöglicht den Austausch des äußeren Zylinders 14 mit äußeren Zylindern die verschiedenartige Schneidfenster aufweisen.

Ein hohler, innerer Zylinder 18 wird in dem äußeren Zylinder 14 axial geführt. Der innere Zylinder 18 weist am ersten Ende eine Öffnung 19 und am zweitem Ende eine Öffnung 20 auf. Im Bereich der Öffnung 19 ist an der Mantelfläche des inneren Zylinders 18 eine erste Schneidöffnung 21 ausgebildet. Der innere Zylinder 18 erstreckt sich vom Bereich des Schneidfensters 16 bis zum dritten pneumatischen Anschluss 13 und ist mit dem Kolben 7 fest verbunden, wodurch der innere Zylinder 18 ebenfalls in eine oszillierende Bewegung versetzt wird.

Der verdrehbare innere Zylinder 18 ist in der Arretiervorrichtung 4 axial verschiebbar, weist aber im Bereich der Öffnung 20 zumindest eine Nase 22 auf, die in eine Nut 23 der Arretiervorrichtung 4 eingreift. Beim Verdrehen der gelösten Arretiervorrichtung 4 wird auch der innere Zylinder 18 verdreht, wodurch es möglich ist zwischen den Betriebsmodi zu wählen. Dafür wird durch Auseinanderziehen des Handgriffs 2 in axialer Richtung die Arretiervorrichtung 4 zum Hauptteil 3 verdrehbar und folglich ist auch der innere Zylinder 18 zum äußeren Zylinder 14 verdrehbar. Nach Einstellen des Betriebsmodus durch Drehung des inneren Zylinders 18 wird dieser durch Arretierung der Arretiervorrichtung 4 wieder gegenüber dem äußeren Zylinder 14 gegen Verdrehen gesichert. In dieser Ausführungsform des erfindungsgemäßen, chirurgischen Handstücks 1 erfolgt die Wahl zwischen den Betriebsmodi durch eine 180°-Verdrehung der Arretiervorrichtung 4.

Am inneren Zylinder 18 ist eine Gewinde 24 vorgesehen, das den Austausch mit inneren Zylindern, die verschiedenartige Schneidöffnungen aufweisen, ermöglicht. Hierdurch ist der Vorteil erhalten, dass unterschiedliche innere Zylinder verwendet werden können, die unterschiedliche Betriebsmodi mit unterschiedlichen Schneidvorgängen ermöglichen.

Es kann erwähnt werden, dass die lösbaren Verbindungen zum Austausch des inneren und äußeren Zylinders auch ohne Gewinde ausgeführt sein können. Des Weiteren kann die Arretiervorrichtung auch so ausgeführt sein, dass nur der innere Zylinder und / oder nur der Kolben gegen Verdrehen gesichert wird.

Figur 2 zeigt den Schnittvorgang des chirurgischen Handstücks 1 gemäß Figur 1 im ersten Betriebsmodus, wobei nur der Ausschnitt B des chirurgischen Handstücks 1 aus Figur 1 dargestellt ist. Die Öffnung 19 ist über den hohlen, inneren Zylinder 18 zur Abfuhr eines abgetrennten, biologischen Gewebes 25 mit dem dritten, pneumatischen Anschluss 13 verbunden. Im ersten Betriebsmodus liegen das Schneidfenster 16 und eine erste Schneidöffnung 21 des inneren Zylinders 18, bezogen auf die Rotationsachsen der Zylinder 14 und 18, auf gegenüberliegenden Seiten. Somit bilden nur das Schneidfenster 16 des äußeren Zylinders 14 und eine Stirnfläche 26 des inneren Zylinders 18 eine Schnittkante 27. Vor dem ersten Schritt des Schnittvorgangs wurde durch das Schneidfenster 16 biologisches Gewebe 28 in den äußeren Zylinder 14 eingesaugt. Danach drängt der sich in Richtung RV vorwärtsbewegende, innere Zylinder 18 das biologische Gewebe 28 in Richtung Schnittkante 27. Durch die Vorwärtsbewegung des inneren Zylinders 18 überfährt die Stirnfläche 26 das Schneidfenster 16 bei dem zweiten Schritt und das biologische Gewebe 28 wird an der Schnittkante 27 abgetrennt. Danach erreicht der innere Zylinder 18 seine vordere Endposition VE und durch eine Rückwärtsbewegung in Richtung RR wird das Schneidfenster 16 von neuem geöffnet. Währenddessen wird das abgetrennte, biologische Gewebe 25 durch Unterdruck über den hohlen, inneren Zylinder 18 abgeführt.

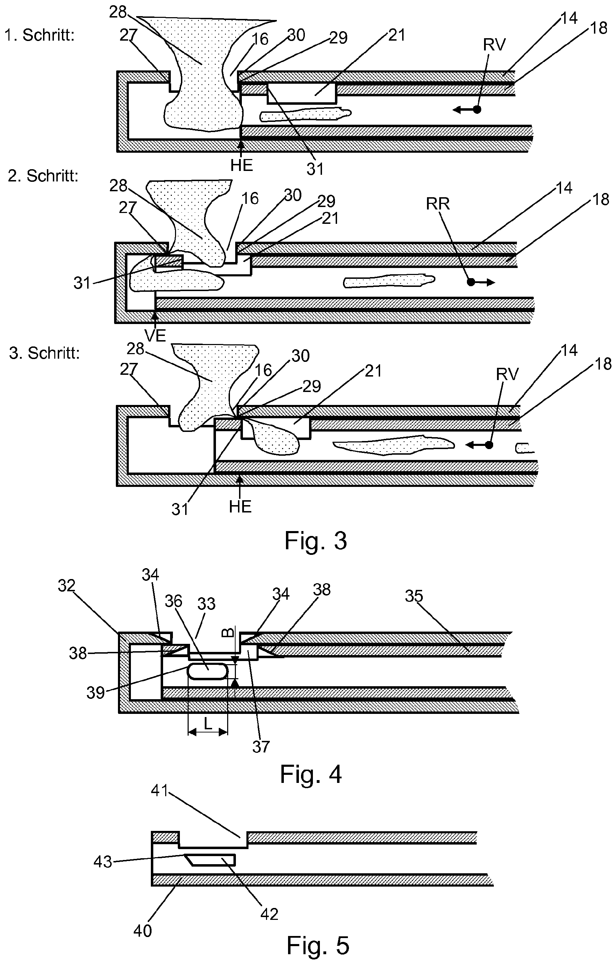

Figur 3 zeigt die Schnittvorgänge des chirurgischen Handstücks aus Figur 1 im zweiten Betriebsmodus, wobei nur Ausschnitt B dargestellt ist. Der Aufbau entspricht dem der Figur 1, wobei durch die Einstellung des zweiten Betriebsmodus der innerer Zylinder 18, im Vergleich zu Figur 1, um 180° gedreht ist. Somit liegen die erste Schneidöffnung 21 des inneren Zylinders 18 und das Schneidfenster 16 des äußeren Zylinders 14, bezogen auf die Rotationsachsen der Zylinder 14 und 18, auf derselben Seite. Dadurch wird im zweiten Betriebsmodus eine weitere Schnittkante 29 gebildet. Der erste Abtrennvorgang gemäß dem zweiten Schritt an der Schnittkante 27 entspricht dem des ersten Betriebsmodus nach Figur 2. Parallel zum ersten Abtrennvorgang werden durch die Vorwärtsbewegung, in Richtung RV, des inneren Zylinders 18 das Schneidfenster 16 und die erste Schneidöffnung 21 übereinander geschoben. Durch diese Öffnung wird biologisches Gewebe 28 für einen zweiten Abtrennvorgang eingesaugt. Der rückwärts in Richtung RR bewegte innere Zylinder 18 drängt bei einem dritten Schritt das eingesaugte biologische Gewebe 28 in Richtung Schnittkante 29. Die Schnittkante 29 entsteht an einer Fläche 30 des Schneidfensters 16, an der sich, durch die Rückwärtsbewegung des inneren Zylinders 18, eine Fläche 31 der ersten Schneidöffnung 21 vorbeibewegt. Währenddessen wird für den nächsten Abtrennvorgang an der Schnittkante 27 biologisches Gewebe 28 durch das Schneidfenster 16 eingesaugt.

Es kann erwähnt werden, dass die Zylinder so ausgeführt werden könnten, dass auch mit den gegenüberliegenden Flächen der Flächen 30 und 31 eine Schnittkante gebildet wird. Hierfür kann es notwendig sein beispielsweise die erste Schneidöffnung 21 in ihrer Länge kürzer auszuführen. Als vorteilhaft hat sich auch erwiesen die Endpositionen HV und HE entlang der Zylinderachsen verschiebbar und fixierbar auszuführen. Hierdurch kann durch Veränderung einer oder beider Endpositionen eine oder mehrere Schnittkanten in Eingriff gebracht werden.

Figur 4 zeigt einen äußeren Zylinder 32, an dem ein Schneidfenster 33 ausgebildet ist, welches Schneidkanten 34 aufweist. Ein innerer Zylinder 35 ist verschiebbar im äußeren Zylinder 32 geführt. Der innere Zylinder 35 weist im Bereich eines Endes an der Mantelfläche eine erste Schneidöffnung 36 und zumindest eine zweite Schneidöffnung 37 auf, die sich in der Breite B und / oder der Länge L zur ersten Schneidöffnung 36 unterscheidet und Schneidkanten 38 aufweist. Durch eine in Figur 4 nicht dargestellte Arretiervorrichtung ist zumindest ein dritter Betriebsmodus einstellbar, bei dem das Schneidfenster 33 des äußeren Zylinders 32 und die zweite Schneidöffnung 37 des inneren Zylinders 35, zur Bildung zumindest einer zusätzlichen Schnittkante, ausgerichtet sind. Die Schnittvorgänge des dritten Betriebsmodus folgen dem Ablauf wie in Figur 3 beschrieben. Eine Schnittkanten bildende Fläche 39 der ersten Schneidöffnung 36 ist konvex ausgeführt. Mit dieser konvexen Form und auch mit den Schneidkanten 34 und 38 wird das biologische Gewebe für den endgültigen Abtrennvorgang auf einen kleinen Bereich der Schnittkante konzentriert, wodurch besonders gute Schnitte erzielbar sind.

Es kann erwähnt werden, dass Schnittkanten bildende Flächen auch konkav ausgeführt sein können. Des Weiteren kann die Hubgeschwindigkeit des inneren Zylinders zum äußeren Zylinder einstellbar sein, wodurch vorteilhafterweise auch die Öffnungsdauer des Schneidfensters einstellbar ist.

Figur 5 zeigt einen inneren Zylinder 40 eines erfindungsgemäßen chirurgischen Handstücks der eine erste Schneidöffnung 41 und eine zweite Schneidöffnung 42 aufweist. Die zweite Schneidöffnung 42 weist einen spitzen Winkel 43 auf. Durch diese Form wird biologisches Gewebe, für den endgültigen Abtrennvorgang, auf einen kleinen Bereich der Schnittkante konzentriert.

## Patentansprüche

1. Chirurgisches Handstück (1) zum Entfernen von biologischem Gewebe umfassend:
• einen hohlen, äußeren Zylinder (14; 32), der an einem Ende (17) geschlossen ist, wobei im Bereich des Endes (17) an der Mantelfläche des äußeren Zylinders (14; 32) ein Schneidfenster (16; 33) ausgebildet ist;
• einen hohlen, inneren Zylinder (18; 35; 40), der an einem ersten Ende und an einem zweiten Ende eine Öffnung (19, 20) aufweist und im äußeren Zylinder (14; 32) mit einem Antrieb, zwischen zwei Endpositionen (VE, HE), koaxial verschiebbar ist, wobei bei einem ersten Betriebsmodus eine Stirnfläche (26) des ersten Endes mit dem Schneidfenster (16; 33) des äußeren Zylinders (14; 32) eine Schnittkante (27) bildet;
wobei der innere Zylinder (18; 35; 40) im Bereich des ersten Endes an der Mantelfläche eine erste Schneidöffnung (21; 36; 41) aufweist und zum äußeren Zylinder (14; 32) verdrehbar ausgebildet ist, **dadurch gekennzeichnet, dass** durch Drehung des inneren Zylinders ein zweiter Betriebsmodus einstellbar ist, bei dem das Schneidfenster (16; 33) des äußeren Zylinders (14; 32) und die erste Schneidöffnung (21; 36; 41) des inneren Zylinders (18; 35; 40), zur Bildung zumindest einer weiteren Schnittkante (29), ausgerichtet sind und wobei eine Arretiervorrichtung (4) vorgesehen ist, die den inneren Zylinder (18; 35; 40) gegenüber dem äußeren Zylinder (14; 32) in den eingestellten Betriebsmodi gegen Verdrehen sichert.

2. Chirurgisches Handstück gemäß Anspruch 1, **dadurch gekennzeichnet, dass** der innere Zylinder (35; 40) im Bereich des ersten Endes an der Mantelfläche zumindest eine zweite Schneidöffnung (37; 42) aufweist, die sich in der Breite (B) und / oder der Länge (L) zur ersten Schneidöffnung (36; 41) unterscheidet, und durch die Arretiervorrichtung zumindest ein dritter Betriebsmodus einstellbar ist, bei dem das Schneidfenster (33) des äußeren Zylinders (32) und die zweite Schneidöffnung (37; 42) des inneren Zylinders (35; 40), zur Bildung zumindest einer zusätzlichen Schnittkante, ausgerichtet sind.

3. Chirurgisches Handstück gemäß einem der vorhergehenden Ansprüche, **dadurch gekennzeichnet, dass** die Schneidöffnung (37) und / oder das Schneidfenster (33) Schneidkanten (34, 38) aufweisen.

4. Chirurgisches Handstück gemäß einem der vorhergehenden Ansprüche, **dadurch gekennzeichnet, dass** die Schneidöffnung (42) und / oder das Schneidfenster spitze Winkeln (43) aufweisen.

5. Chirurgisches Handstück gemäß einem der vorhergehenden Ansprüche, **dadurch gekennzeichnet, dass** zumindest eine der die Schnittkanten bildenden Flächen (39) konkav und / oder konvex ausgeführt sind.

6. Chirurgisches Handstück gemäß einem der vorhergehenden Ansprüche, **dadurch gekennzeichnet, dass** die Endpositionen (VE, HE) entlang der Zylinderachse verschiebbar und fixierbar sind.

7. Chirurgisches Handstück gemäß einem der vorhergehenden Ansprüche, **dadurch gekennzeichnet, dass** eine Hubgeschwindigkeit des inneren Zylinders zum äußeren Zylinder einstellbar ist.

8. Chirurgisches Handstück gemäß einem der vorhergehenden Ansprüche, **dadurch gekennzeichnet, dass** der äußere Zylinder (14) und / oder der innere Zylinder (18), mit Zylindern die verschiedenartige Schneidfenster und / oder Schneidöffnungen aufweisen, austauschbar sind.

## Claims

1. A surgical hand-held tool (1) for removing biological tissue, comprising:
• a hollow external cylinder (14; 32), which is closed at one end (17), wherein a cutting window (16; 33) is formed in the region of the end (17) at the shell surface of the external cylinder (14; 32);
• a hollow internal cylinder (18; 35; 40), which has an opening (19, 20) at a first end and at a second end and may be coaxially shifted within the external cylinder (14; 32) using a drive between two end positions (VE, HE), wherein in a first operational mode a front face (26) of the first end forms a cutting edge (27) with the cutting window (16; 33) of the external cylinder (14; 32);
wherein the internal cylinder (18; 35; 40) in the region of the first end at the shell surface has a first cutting opening (21; 36; 41) and is configured to be rotatable towards the external cylinder (14; 32), **characterized in that** by rotation of the internal cylinder there may be set a second operational mode, wherein the cutting window (16; 33) of the external cylinder (14; 32) and the first cutting opening (21; 36; 41) of the internal cylinder (18; 35; 40) are configured to form a further cutting edge (29) and wherein a locking device (4) is provided, which secures the internal cylinder (18; 35; 40) in regard to the external cylinder (14; 32) in the set operational modes against rotation.

2. A surgical hand-held tool according to claim 1, **characterized in that** the internal cylinder (35; 40) in the region of the first end at the shell surface has at least one second cutting opening (37; 42), which differs from the first cutting opening (36; 41) in width (B) and/or length (L), and that at least one third operational mode may be set by the locking device, wherein the cutting window (33) of the external cylinder (32) and the second cutting opening (37; 42) of the internal cylinder (35, 40) are configured to form at least one additional cutting edge.

3. A surgical hand-held tool according to any of the preceding claims, **characterized in that** the cutting opening (37) and/or the cutting window (33) have cutting edges (34, 38)

4. A surgical hand-held tool according to any of the preceding claims, **characterized in that** the cutting opening (42) and/or the cutting window have acute angles (43).

5. A surgical hand-held tool according to any of the preceding claims, **characterized in that** at least one of the surfaces (39) forming the cutting edges is configured to be concave and/or convex.

6. A surgical hand-held tool according to any of the preceding claims, **characterized in that** the end positions (VE, HE) may be shifted and fixed along the cylinder axis.

7. A surgical hand-held tool according to any of the preceding claims, **characterized in that** a stroke rate of the internal cylinder in regard to the external cylinder may be set.

8. A surgical hand-held tool according to any of the preceding claims, **characterized in that** the external cylinder (14) and/or the internal cylinder (18) may be replaced for cylinders having different cutting windows and/or cutting openings.

## Revendications

1. Pièce à main chirurgicale (1) permettant d'enlever du tissu biologique, comprenant:
- un cylindre extérieur creux (14; 32), qui est fermé à une extrémité (17), dans laquelle une fenêtre de coupe (16; 33) est formée dans la face latérale du cylindre extérieur (14; 32) dans la région de l'extrémité (17);
- un cylindre intérieur creux (18; 35; 40), qui présente une ouverture (19, 20) à une première extrémité et à une seconde extrémité et qui peut être déplacé de façon coaxiale dans le cylindre extérieur (14; 32) avec un entraînement, entre deux positions d'extrémité (VE, HE), dans laquelle dans un premier mode de fonctionnement une face frontale (26) de la première extrémité forme avec la fenêtre de coupe (16; 33) du cylindre extérieur (14; 32) une arête de coupe (27);
dans laquelle le cylindre intérieur (18; 35; 40) présente dans la région de la première extrémité dans la face latérale une première ouverture de coupe (21; 36; 41) et peut tourner par rapport au cylindre extérieur (14; 32),
**caractérisée en ce que**, par la rotation du cylindre intérieur, on peut régler un second mode de fonctionnement, dans lequel la fenêtre de coupe (16; 33) du cylindre extérieur (14; 32) et la première ouverture de coupe (21; 36; 41) du cylindre intérieur (18; 35; 40) sont alignées pour former au moins une autre arête de coupe (29), et dans laquelle il est prévu un dispositif de blocage (4), qui immobilise le cylindre intérieur (18; 35; 40) par rapport au cylindre extérieur (14; 32) contre une rotation dans les modes de fonctionnement réglés.

2. Pièce à main chirurgicale selon la revendication 1, **caractérisée en ce que** le cylindre intérieur (35; 40) présente dans la région de la première extrémité dans la face latérale au moins une deuxième ouverture de coupe (37; 42), qui se différencie de la première ouverture de coupe (36; 41) par la largeur (B) et/ou par la longueur (L), et au moins un troisième mode de fonctionnement peut être réglé par le dispositif de blocage, dans lequel la fenêtre de coupe (33) du cylindre extérieur (32) et la deuxième ouverture de coupe (37; 42) du cylindre intérieur (35; 40) sont alignées pour former au moins une arête de coupe supplémentaire.

3. Pièce à main chirurgicale selon l'une quelconque des revendications précédentes, **caractérisée en ce que** l'ouverture de coupe (37) et/ou la fenêtre de coupe (33) présentent des arêtes de coupe (34, 38).

4. Pièce à main chirurgicale selon l'une quelconque des revendications précédentes, **caractérisée en ce que** l'ouverture de coupe (42) et/ou la fenêtre de coupe présentent des angles aigus (43).

5. Pièce à main chirurgicale selon l'une quelconque des revendications précédentes, **caractérisée en ce qu'**au moins une des faces (39) formant les arêtes de coupe est de forme concave et/ou convexe.

6. Pièce à main chirurgicale selon l'une quelconque des revendications précédentes, **caractérisée en ce que** les positions d'extrémité (VE, HE) peuvent être déplacées et fixées le long de l'axe des cylindres.

7. Pièce à main chirurgicale selon l'une quelconque des revendications précédentes, **caractérisée en ce qu'**une vitesse de course du cylindre intérieur par rapport au cylindre extérieur est réglable.

8. Pièce à main chirurgicale selon l'une quelconque des revendications précédentes, **caractérisée en ce que** le cylindre extérieur (14) et/ou le cylindre intérieur (18) peuvent être échangés pour des cylindres, qui présentent des fenêtres de coupe et/ou des ouvertures de coupe de nature différente.
